(19)

Europäisches Patentamt
European Patent Office
Office européen des brevets

(11) **EP 4 574 116 A1**

(12) **EUROPEAN PATENT APPLICATION**
published in accordance with Art. 153(4) EPC

(43) Date of publication:
**25.06.2025 Bulletin 2025/26**

(21) Application number: **22961028.2**

(22) Date of filing: **30.09.2022**

(51) International Patent Classification (IPC):
**A61F 13/15** (2006.01)    **A61F 13/532** (2006.01)
**A61F 13/533** (2006.01)    **A61F 13/536** (2006.01)

(52) Cooperative Patent Classification (CPC):
**A61F 13/536; A61F 13/15; A61F 13/15617;**
**A61F 13/15707; A61F 13/532; A61F 13/533;**
A61F 2013/530481

(86) International application number:
**PCT/JP2022/036806**

(87) International publication number:
**WO 2024/069969 (04.04.2024 Gazette 2024/14)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Unicharm Corporation**
**Shikokuchuo-shi, Ehime 799-0111 (JP)**

(72) Inventors:
• **ONISHI, Kazuaki**
  **Kanonji-shi, Kagawa 769-1602 (JP)**
• **TSUKUDA, Atsushi**
  **Kanonji-shi, Kagawa 769-1602 (JP)**
• **SASAYAMA, Kenichi**
  **Kanonji-shi, Kagawa 769-1602 (JP)**
• **KONDO, Daiki**
  **Kanonji-shi, Kagawa 769-1602 (JP)**

(74) Representative: **Staeger & Sperling**
**Partnerschaftsgesellschaft mbB**
**Sonnenstraße 19**
**80331 München (DE)**

(54) **ABSORBER FOR ABSORBENT ARTICLE, AND METHOD FOR MANUFACTURING SAID ABSORBER**

(57)    The purpose of the present disclosure is to provide an absorber having an exceptional fit when worn and having exceptional absorption properties. An absorber according to the present disclosure has the following configuration. An absorber (7) including an integrally formed absorption core (9) for an absorbent article, the absorption core (9) including pulp fiber (39) and super-absorbent polymer particles (37), wherein the absorber (7) is characterized in that: the absorption core (9) is provided with a narrow-width part (35) that has a low length in the width direction (W) and that is disposed in a crotch region (19); in the narrow-width part (35) in the crotch region (19), the absorption core (9) is provided with a plurality of crotch region grooves (41) that extend along the longitudinal direction (L) and that are set apart from each other in the width direction (W); and in the absorption core (9), the average basis weight of the super-absorbent polymer particles (37) in the crotch region (19) is higher than the average basis weight of the super-absorbent polymer particles (37) in the absorption core (9) as a whole.

Fig. 3

EP 4 574 116 A1

**Description**

FIELD

The present disclosure relates to an absorbent body for an absorbent article and a method of producing the absorbent body.

BACKGROUND

Studies are being conducted to improve absorbency in an absorbent core of an absorbent article containing pulp fibers and super absorbent polymer particles by providing grooves and changing the ratio of pulp fibers and super absorbent polymer particles around the grooves.

For example, Patent Literature 1 discloses an absorbent article which comprises an absorbent core, which has a longitudinal direction, a transverse direction, and a thickness direction and which contains super absorbent polymer particles, wherein the absorbent core has a non-skin-facing surface and a skin-facing surface, and is composed of a first layer on the non-skin-facing surface side and a second layer on the skin-facing surface side, the first layer extends along the longitudinal direction and comprises a plurality of grooves including a plurality of main grooves penetrating in the thickness direction, and a plurality of base parts extending along the longitudinal direction, each of the main grooves and each of the base parts extends alternatingly in the transverse direction, the second layer comprises a plurality of parts corresponding to the main grooves and a plurality of parts corresponding to the base parts, respectively, at positions overlapping the main grooves and the base parts in the thickness direction, and the average density of the super absorbent polymer particles contained in each of the plurality of base parts and the average density of the super absorbent polymer particles contained in each of the plurality of parts corresponding to the main grooves are both lower than the average density of the super absorbent polymer particles contained in each of the plurality of parts corresponding to the base parts.

Furthermore, Patent Literature 2 discloses an absorbent article which comprises an absorbent core, which has a longitudinal direction, a transverse direction, and a thickness direction, and which contains super absorbent polymer particles, wherein the absorbent core has a non-skin-facing surface and a skin-facing surface, and is composed of a first layer on the non-skin-facing surface side and a second layer on the skin-facing surface side, wherein the first layer extends along the longitudinal direction and comprises a plurality of grooves including a plurality of main grooves penetrating in the thickness direction, and a plurality of base parts extending along the longitudinal direction; each of the plurality of main grooves and each of the plurality of base parts extend alternatingly in the transverse direction, and each of the plurality of base parts comprises a base part inner part adjacent to the non-skin-facing surface side of the second layer and a base part outer part adjacent to the non-skin-facing surface side of the base part inner part, and the average density of the super absorbent polymer particles contained in the base part outer part is lower than the average density of the super absorbent polymer particles contained in the base part inner part.

[Citation List]

[Patent Literature]

[PTL 1]
Japanese Unexamined Patent Publication (Kokai) No. 2018-000872
[PTL 2]
Japanese Unexamined Patent Publication (Kokai) No. 2021-053237

SUMMARY

[Technical Problem]

In the absorbent cores according to Patent Literature 1 and Patent Literature 2, the average density of the super absorbent polymer particles around the grooves (main grooves) is adjusted to improve repeated absorption performance.

However, since the absorbent cores in Patent Literature 1 and Patent Literature 2 are each integrally molded, it is difficult to change the average basis weight of the super absorbent polymer particles in the longitudinal region of the absorbent core, and from this perspective there is room for improvement.

Thus, an object of the present disclosure is to provide an absorbent body comprising an integrally molded absorbent core which provides excellent fit when worn as well as excellent absorbency.

[Solution to Problem]

The present inventors have discovered an absorbent body for an absorbent article, comprising an integrally molded absorbent core, the absorbent core comprising pulp fibers and super absorbent polymer particles, and having mutually perpendicular longitudinal, width, and thickness directions, wherein the absorbent core is partitioned in the longitudinal direction into an abdomen-side region, a crotch region, and a back-side region, in order from the front, and comprises a narrow part which is arranged in the crotch region and has a short length in the width direction, the absorbent core comprises a plurality of crotch region grooves in the narrow part in the crotch region which extend along the longitudinal direction and which are spaced apart in the width direction, and an average basis weight of the super absorbent polymer particles in the crotch region in the absorbent core is higher than an average basis weight of the super absorbent polymer particles in the entirety of the absorbent core.

[Advantageous Effects of Invention]

The absorbent body including the integrally molded absorbent core of the present disclosure has excellent fit when worn and excellent absorbency.

BRIEF DESCRIPTION OF DRAWINGS

FIG. 1 is a view detailing an absorbent body 7 according to a first embodiment.
FIG. 2 is a view detailing the absorbent body 7 according to the first embodiment.
FIG. 3 is a view detailing the absorbent body 7 according to the first embodiment.
FIG. 4 is a view detailing the absorbent body 7 according to the first embodiment.
FIG. 5 is a view detailing an absorbent body according to a second embodiment.
FIG. 6 is a view detailing an absorbent body 7 according to a third embodiment.
FIG. 7 is a view detailing a method of producing an absorbent core 9 according to the first embodiment.
FIG. 8 is a view detailing a method of producing the absorbent core 9 according to the first embodiment.
FIG. 9 is a view detailing Examples.
FIG. 10 is a view detailing Examples.
FIG. 11 is a view detailing Examples.

DESCRIPTION OF EMBODIMENTS

Specifically, the present disclosure relates to the following aspects.

[Aspect 1]

An absorbent body for an absorbent article, comprising an integrally molded absorbent core, the absorbent core comprising pulp fibers and super absorbent polymer particles, and having mutually perpendicular longitudinal, width, and thickness directions, wherein

the absorbent core is partitioned in the longitudinal direction into an abdomen-side region, a crotch region, and a back-side region, in order from a front, and comprises a narrow part which is arranged in the crotch region and has a short length in the width direction,
the absorbent core comprises a plurality of crotch region grooves in the narrow part in the crotch region which extend along the longitudinal direction and which are spaced apart in the width direction, and
an average basis weight of the super absorbent polymer particles in the crotch region in the absorbent core is higher than an average basis weight of the super absorbent polymer particles in the entirety of the absorbent core.

In the absorbent body described above, the absorbent core comprises a plurality of crotch region grooves in the narrow part of the crotch region. In general, a narrow part provided with a plurality of crotch region grooves has an advantage in that liquid such as bodily fluid excreted in the narrow part can be diffused in the longitudinal direction through the plurality of crotch region grooves.
However, in the narrow part, since the absorbent core is narrow and comprises a plurality of crotch region grooves, the absolute amount of the absorbent core is small. As the absorbent core repeatedly absorbs liquid which reaches the narrow part in the narrow part, liquid which cannot be held thereby may leak from the narrow part.

Conversely, if the absolute amount of the narrow part is increased in order to improve the absorbency of the narrow part, the average basis weight of the narrow part increases, the thickness of the narrow part increases, and the rigidity of the narrow part increases, which leads to a decrease in initial fit. Further, as the absorbent core repeatedly absorbs liquid which reaches the narrow part in the narrow part having a high average basis weight, the liquid is locally retained in the narrow part, whereby the spaces between the plurality of crotch region grooves are reduced, and the narrow part becomes thicker, which tends to reduce wearing comfort over time.

In the absorbent core of the absorbent body according to Aspect 1, the average basis weight of the super absorbent polymer particles in the crotch region is higher than the average basis weight of the super absorbent polymer particles in the entirety of the absorbent core. The super absorbent polymer particles held together with the pulp fibers are unlikely to give the wearer a feeling of stiffness, and thus, the absorbent body has excellent fit when worn (initial and over time).

Furthermore, since the super absorbent polymer particles have a slower liquid absorption rate than the pulp fibers, liquid which reaches the narrow part can be diffused in the longitudinal direction through the crotch region grooves. Thus, the absorbency of the absorbent body is unlikely to be reduced even if liquid is absorbed repeatedly.

[Aspect 2]

The absorbent body according to Aspect 1, wherein in the absorbent core, a ratio of the super absorbent polymer particles in the crotch region is higher than a ratio of the super absorbent polymer particles in the back-side region.

In the absorbent core of the absorbent body described above, the ratio of super absorbent polymer particles in the crotch region is higher than that in the back-side region. Thus, the integrally molded absorbent body has an excellent fit when worn, and the water storage capacity of the plurality of crotch region grooves is less likely to be reduced, whereby the liquid retention capacity of the crotch region is high and the absorbency is less likely to be reduced even when liquid is repeatedly absorbed.

[Aspect 3]

The absorbent body according to Aspect 1 or 2, wherein in the absorbent core, a ratio of the super absorbent polymer particles in the crotch region is higher than a ratio of the super absorbent polymer particles in the abdomen-side region.

In the absorbent body described above, the ratio of super absorbent polymer particles in the crotch region is higher than that in the abdomen-side region. Thus, the integrally molded absorbent body has an excellent fit when worn, and the water storage capacity of the plurality of crotch region grooves is less likely to be reduced, whereby the liquid retention capacity of the crotch region is high and the absorbency is less likely to be reduced even when liquid is repeatedly absorbed.

[Aspect 4]

The absorbent body according to any one of Aspects 1 to 3, wherein the absorbent core comprises a skin-facing surface and a non-skin-facing surface, and also comprises a first layer and a second layer which are adjacent to each other in the thickness direction,

the first layer has a plurality of through hole parts which are arranged in the crotch region and which correspond to the plurality of crotch region grooves, and base parts which are arranged between the plurality of through hole parts, the second layer has a plurality of parts corresponding to the through hole parts and parts corresponding to the base parts, respectively, at positions overlapping the plurality of through hole parts and the base parts in the thickness direction, and in the base parts or the parts corresponding to the base parts constituting the skin-facing surface, an average density of the super absorbent polymer particles is higher than an average density of the super absorbent polymer particles in the parts corresponding to the through hole parts.

When the first layer constitutes the skin-facing surface and the base parts and the through hole parts constitute the skin-facing surface, in the absorbent body described above, the average density of the super absorbent polymer particles in the base parts constituting the skin-facing surface is higher than the average density of the super absorbent polymer particles in the parts corresponding to the through hole parts constituting the non-skin-facing surface.

Specifically, the average density of the super absorbent polymer particles is as follows:
Base parts (first layer) > parts corresponding to through hole parts (second layer) > through hole parts (first layer)

Thus, the through hole parts (crotch region grooves) of the first layer, which have the lowest density (near 0) of the super absorbent polymer particles, can receive the liquid and diffuse it in the longitudinal direction at a predetermined flow rate, and the liquid received by the through hole parts of the first layer can be transferred to the parts corresponding to the through holes of the second layer, which is adjacent in the thickness direction. In the parts corresponding to the through holes of the second layer, which have a relatively higher average density of the super absorbent polymer particles than the through hole parts of the first layer, the liquid can be diffused in the longitudinal direction at a predetermined rate slower than that of the through hole parts of the first layer.

Next, the liquid received by the parts corresponding to the through holes of the second layer can then be transferred to the parts corresponding to the base parts of the second layer adjacent in the width direction, and also to the base parts of the first layer adjacent in the thickness direction to the parts corresponding to the base parts of the second layer. The base parts of the first layer have a higher average density of the super absorbent polymer particles than the parts corresponding to the through holes of the second layer, and thus, have excellent liquid retention. As a result, the absorbent body has high liquid retention in the crotch region, whereby absorbency is unlikely to be reduced even when liquid is repeatedly absorbed.

When the second layer constitutes the skin-facing surface and the parts corresponding to the base parts and the parts corresponding to the through holes constitute the skin-facing surface, in the absorbent body described above, the average density of the super absorbent polymer particles in the parts corresponding to the base parts constituting the skin-facing surface is higher than the average density of the super absorbent polymer particles in the parts corresponding to the through hole parts constituting the skin-facing surface.

Specifically, the average density of super absorbent polymer particles is as follows:

Parts corresponding to base parts (second layer) > parts corresponding to through holes (second layer) > through hole parts (first layer)

Thus, liquid can pass through the parts corresponding to through hole parts of the second layer, which have a relatively low average density of the super absorbent polymer particles, and be moved to the through hole parts of the first layer, which have the lowest average density of the super absorbent polymer particles, and the liquid can be diffused in the longitudinal direction. The liquid received by the through hole parts can then be transferred to the base parts adjacent in the width direction, and to the parts corresponding to the base parts of the second layer adjacent in the thickness direction to the base parts of the first layer. The parts corresponding to the base parts of the second layer have a higher average density of the super absorbent polymer particles than the parts corresponding to the through holes of the second layer, and thus, are excellent in liquid retention. As a result, the absorbent body has high liquid retention in the crotch region, whereby absorbency is unlikely to be reduced even when liquid is repeatedly absorbed.

[Aspect 5]

The absorbent body according to Aspect 4, wherein an average density of the super absorbent polymer particles in the base parts or the parts corresponding to the base parts constituting the skin-facing surface is higher than an average density of the super absorbent polymer particles in the base parts or the parts corresponding to the base parts constituting the non-skin-facing surface.

When the first layer constitutes the skin-facing surface and the base parts and the parts corresponding to the through holes constitute the skin-facing surface, in the absorbent body described above, the average density of the super absorbent polymer particles in the base parts constituting the skin-facing surface is higher than the average density of the super absorbent polymer particles in the parts corresponding to the base parts constituting the non-skin-facing surface.

Specifically, the average density of super absorbent polymer particles is as follows:

Base parts (first layer) > parts corresponding to through holes (second layer) > through hole parts (first layer)
Base parts (first layer) > parts corresponding to base parts (second layer)

Thus, liquid received by the parts corresponding to the base parts of the second layer, in which the average density of the super absorbent polymer particles is relatively low, can be diffused in the longitudinal direction and transferred to the base parts of the first layer adjacent to the parts corresponding to the base parts of the second layer in the thickness direction. The base parts of the first layer have excellent liquid retention because the average density of the super absorbent polymer particles therein is higher than that of the parts corresponding to the base parts of the second layer. As a result, the absorbent body has high liquid retention in the crotch region, whereby absorbency is unlikely to be reduced even when liquid is repeatedly absorbed.

When the second layer constitutes the skin-facing surface and the parts corresponding to the base parts and the parts corresponding to the through holes constitute the skin-facing surface, in the absorbent body described above, the average density of the super absorbent polymer particles in the parts corresponding to the base parts constituting the skin-facing surface is higher than the average density of the super absorbent polymer particles in the parts corresponding to the through hole parts constituting the skin-facing surface.

EP 4 574 116 A1

Specifically, the average density of super absorbent polymer particles is as follows:

Parts corresponding to base parts (second layer) > parts corresponding to through holes (second layer) > parts corresponding to through holes (first layer)

Parts corresponding to base parts (second layer) > base parts (first layer)

Thus, liquid received by the base parts of the first layer, in which the average density of the super absorbent polymer particles is relatively low, can be diffused in the longitudinal direction and transferred to the parts corresponding to the base parts of the second layer adjacent in the thickness direction to the base parts of the first layer. The parts corresponding to the base parts of the second layer have a higher average density of the super absorbent polymer particles than the base parts of the first layer, and thus, have excellent liquid retention. As a result, the absorbent body has high liquid retention in the crotch region, whereby absorbency is unlikely to be reduced even when liquid is repeatedly absorbed.

[Aspect 6]

The absorbent body according to any one of Aspects 1 to 5, wherein in the absorbent core, an average basis weight of the super absorbent polymer particles in the abdomen-side region is lower than an average basis weight of the super absorbent polymer particles in the entirety of the absorbent core.

In the absorbent body described above, the average basis weight of the super absorbent polymer particles in the abdomen-side region is lower than the average basis weight of the super absorbent polymer particles in the entirety of the absorbent core. Thus, even if the absorbent core repeatedly absorbs liquid, the thickness of the absorbent core in the abdomen-side region is unlikely to increase, whereby the absorbent article including the above absorbent core has excellent wearing comfort in the portion (abdomen-side portion) corresponding to the abdomen-side region of the absorbent core.

[Aspect 7]

The absorbent body according to any one of Aspects 1 to 6, wherein in the absorbent core, an average basis weight of the super absorbent polymer particles in the back-side region is lower than an average basis weight of the super absorbent polymer particles in the entirety of the absorbent core.

In the absorbent body described above, the average basis weight of the super absorbent polymer particles in the back-side region is lower than the average basis weight of the super absorbent polymer particles in the entirety of the absorbent core. Thus, even if the absorbent core repeatedly absorbs liquid, the thickness of the absorbent core in the back-side region is unlikely to increase, whereby the absorbent article including the above absorbent core has excellent wearing comfort in the portion (back-side portion) corresponding to the back-side region of the absorbent core.

[Aspect 8]

The absorbent body according to any one of Aspects 1 to 7, wherein at least a part of the plurality of crotch region grooves further comprises a plurality of crotch region sub-grooves which extend in a direction along the width direction.

In the absorbent body described above, since at least a part of the plurality of crotch region grooves further comprises a plurality of crotch region sub-grooves which extend in a direction along the width direction, the volume and surface area of the crotch region grooves can be increased, and thus, liquid can be easily diffused in the longitudinal direction and moved in the width direction.

[Aspect 9]

The absorbent body according to any one of Aspects 1 to 8, wherein the absorbent body comprises an additional absorbent core which is stacked in the thickness direction in a region overlapping the narrow part in the crotch region of the absorbent core in the thickness direction and which has an average basis weight lower than the absorbent core.

In general, when an absorbent core comprises a plurality of grooves in a narrow part, the absorbent core may rupture at the grooves as it absorbs liquid. Furthermore, in general, when an absorbent core has a region in which the average basis weight of super absorbent polymer particles is higher than the average basis weight of super absorbent polymer particles in the entirety of the absorbent core, the absorbent core may rupture at that region as it absorbs liquid.

Since the above absorbent body has a predetermined additional absorbent core in the narrow part in the crotch region, the absorbent body is less likely to rupture during use.

In general, when a plurality of absorbent cores of different sizes, for example, a large absorbent core having a large area and a small absorbent core having a small area, are stacked, the large absorbent core tends to rupture at the boundary (the portion of the large absorbent core that contacts the outer edge of the small absorbent core). Since the above absorbent core has a plurality of crotch region grooves in the crotch region, the plurality of crotch region grooves tend to suppress rupture of the absorbent core at the boundary.

[Aspect 10]

The absorbent body according to any one of Aspects 1 to 9, wherein the absorbent core is partitioned in order from a front, into six equal parts including a first region through a sixth region, the abdomen-side region is constituted by the first region, the crotch region is constituted by the second region and the third region, and the back-side region is constituted by a fourth region through the sixth region.

In the absorbent body described above, since the abdomen-side region, the crotch region, and the back-side region are constituted by the predetermined first region through sixth region, the effects of Aspect 1 can be more effectively exerted.

[Aspect 11]

A method of producing the absorbent body according to Aspect 1, comprising:

a preparation step of preparing a mold member comprising an abdomen-side part, a crotch part, and a back-side part and a narrowed part and a plurality of crotch part ridges, which respectively correspond to the abdomen-side region, the crotch region, and the back-side region and the narrow part and the plurality of crotch region grooves of the absorbent core,

wherein in the mold member, each of the abdomen-side part, the crotch part, and the back-side part has a plurality of openings, each of the plurality of ridges has no openings, and an opening ratio of the crotch part is higher than an opening ratio of each of the abdomen-side part and the back-side part, and

an absorbent core formation step of sprinkling pulp fibers and super absorbent polymer particles from one side of the mold member while reducing pressure from the other side of the mold member to deposit the pulp fibers and the super absorbent polymer particles on the mold member and form the absorbent core.

The above method enables the production of an absorbent body exhibiting the effects of Aspect 1.

[Aspect 12]

A method of producing the absorbent body according to Aspect 8, comprising:

a preparation step of preparing a mold member comprising an abdomen-side part, a crotch part, and a back-side part and a narrowed part, a plurality of crotch part ridges, and a plurality of groin part sub-ridges, which respectively correspond to the abdomen-side region, the crotch region, and the back-side region and the narrow part, the plurality of crotch region grooves, and the plurality of crotch region sub-grooves of the absorbent core,

wherein in the mold member, each of the abdomen-side part, the crotch part, and the back-side part has a plurality of openings, each of the plurality of crotch part ridges and the plurality of crotch part sub-grooves has no openings, and an opening ratio of the crotch part is higher than an opening ratio of each of the abdomen-side part and the back-side part, and

an absorbent core formation step of sprinkling pulp fibers and super absorbent polymer particles from one side of the mold member while reducing pressure from the other side of the mold member to deposit the pulp fibers and the super absorbent polymer particles on the mold member and form the absorbent core.

The above method enables the production of an absorbent body exhibiting the effects of Aspect 8.

[Aspect 13]

The method according to Aspect 11 or 12, wherein the abdomen-side part and the back-side part comprise a plurality of abdomen-side ridges and a plurality of back-side ridges, respectively, extending in the longitudinal direction, and a length in the width direction of each of the plurality of ridges is shorter than a length in the width direction of each of the plurality of abdomen-side ridges and the plurality of back-side ridges.

In the method described above, the length (width) of the width direction of the plurality of ridges in the crotch part is shorter than the width of the plurality of abdomen-side ridges in the abdomen-side part and the width of the plurality of back-side ridges in the back-side part. Thus, the opening ratio of the openings can be increased while forming plurality of crotch region grooves in the crotch part, which makes it easier to produce the absorbent body according to Aspect 1.

[Definitions]

·"Longitudinal direction axis" and "Width direction axis"

In the present description, the term "longitudinal direction axis" refers to the axis extending in the longitudinal direction in the width direction center of the absorbent body or absorbent core. Furthermore, in the present description, the term "width direction axis" refers to the axis extending in the width direction in the longitudinal direction center of the absorbent body or absorbent core.

·"Front" and "Rear"

In the present description, "front" and "rear" refer to the longitudinal directions toward the front and rear of the wearer, respectively.
As used herein, "groove" means a groove extending along the longitudinal direction, and the groove is arranged such that the axis of the groove forms an angle with the longitudinal axis, in an arbitrary position, of preferably less than 45°, more preferably less than 30°, and further preferably less than 15°. When the angle is 45° or more, bodily fluids present in the groove are less likely to flow in the longitudinal direction of the absorbent core.
In the present description, the width of a groove means the length of the groove in the direction perpendicular to the axis of the groove.
In the present description, this groove portion may be referred to as the "main groove" to distinguish it from the sub-groove described below.
In the present description, the term "sub-groove" refers to a groove that is present at a predetermined interval in a direction intersecting with the groove (main groove) and communicates with the groove (main groove) via the base end thereof. The sub-groove is arranged such that the axis of the sub-groove forms an angle of preferably 45° or more, more preferably 60° or more, and further preferably 80° or more with the axis of the groove (main groove) at the portion (i.e., base end) which communicates with the main groove. The sub-groove can extend linearly or non-linearly, for example, curvedly.
In the present description, the width of a sub-groove means the length of the sub-groove in a direction perpendicular to the axis of the sub-groove.
The absorbent body for an absorbent article of the present disclosure and the method of producing the absorbent body will be described in detail below.

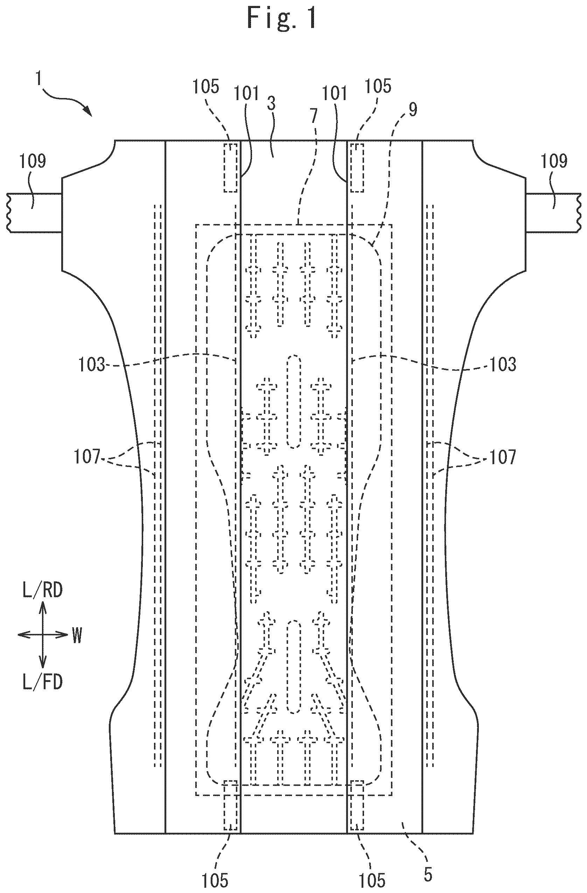

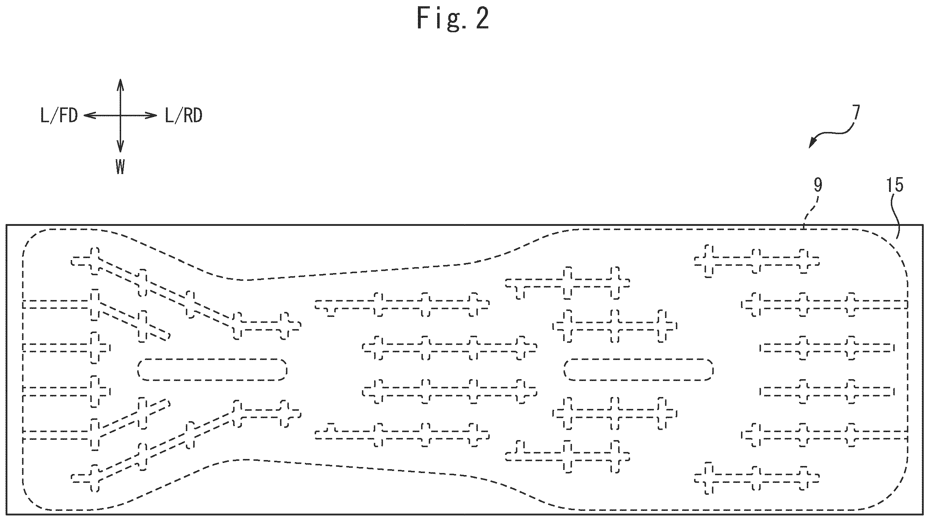

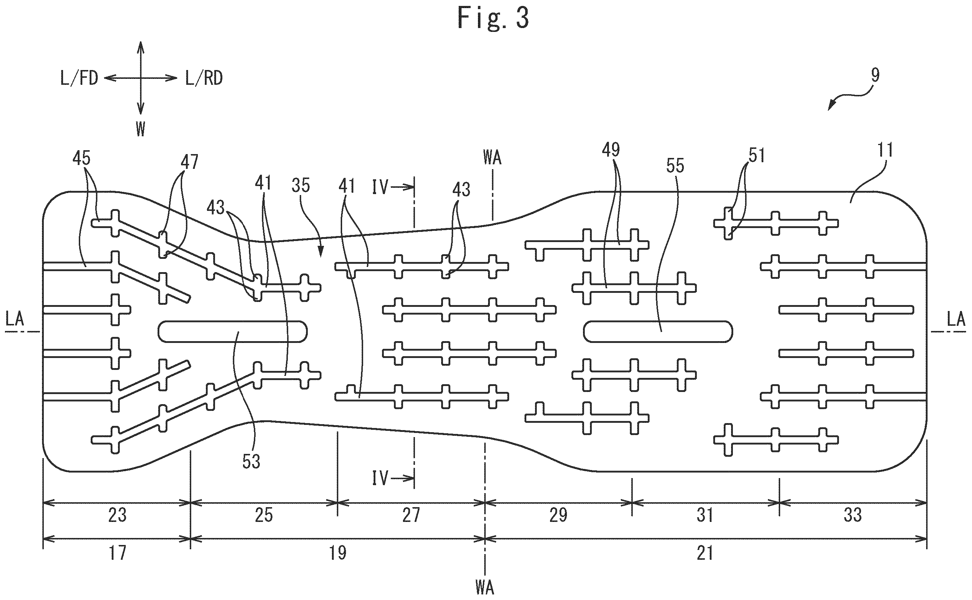

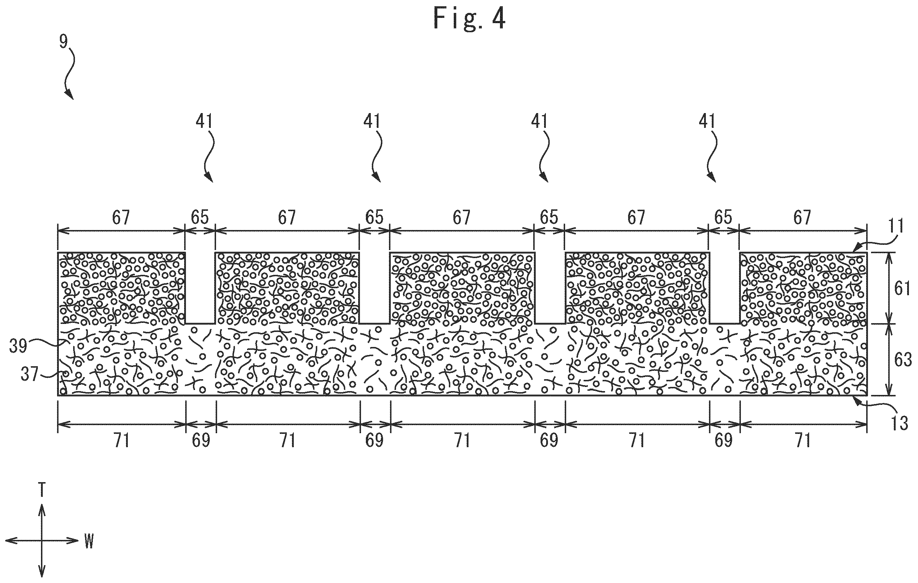

FIGS. 1 to 4 are views detailing an absorbent article, specifically, a disposable diaper 1, including an absorbent body 7 according to an embodiment (hereinafter, sometimes referred to as the "first embodiment") of the present disclosure. Specifically, FIG. 1 is a plan view of the disposable diaper 1. FIG. 2 is a plan view of the absorbent body 7 of the disposable diaper 1. FIG. 3 is a plan view of the absorbent core 9 of the disposable diaper 1. FIG. 4 is an end view at the IV-IV end face of FIG. 3.
The disposable diaper 1 has a longitudinal direction L including a front FD and a rear RD, a width direction W, and a thickness direction T.
The disposable diaper 1 comprises a liquid permeable sheet 3, a liquid impermeable sheet 5, and an absorbent body 7 between the liquid permeable sheet 3 and the liquid impermeable sheet 5.
As shown in FIG. 1, the disposable diaper 1 has a pair of leak-proof walls 101 including elastic members 103, affixation parts 105 for affixation of the leak-proof walls 101 to the liquid permeable sheet 3, elastic members 107, tape fasteners 109, etc., but since these are well known in the relevant technical field, described thereof has been omitted.
The absorbent body 7 has a longitudinal direction L, width direction W, and thickness direction T which are perpendicular to each other in the same manner as the disposable diaper 1, and is composed of an absorbent core 9 having a skin-facing surface 11 and a non-skin-facing surface 13, and a core wrap 15 composed of tissue which covers the skin-facing surface 11 and the non-skin-facing surface 13 of the absorbent core 9.
The absorbent core 9 is integrally molded and contains super absorbent polymer particles 37 and pulp fibers 39. The absorbent core 9 has a longitudinal direction L, width direction W, and thickness direction T which are perpendicular to each other in the same manner as the disposable diaper 1, and the absorbent core 9 has a longitudinal direction axis LA which passes through the width direction W center of the absorbent core 9 and extends parallel to the longitudinal direction L of the absorbent core 9, and a width direction axis WA which passes through the longitudinal direction L center of the absorbent core 9 and extends parallel to the width direction W of the absorbent core 9.
The absorbent core 9 is partitioned in the longitudinal direction L from the front FD to the rear RD into an abdomen-side region 17, a crotch region 19, and a back-side region 21. When the absorbent core 9 is divided into six equal parts in the longitudinal direction L and partitioned from the front into a first region 23, a second region 25, a third region 27, a fourth region 29, a fifth region 31, and a sixth region 33, the abdomen-side region 17 is constituted by the first region 23, the crotch region 19 is constituted by the second region 25 and the third region 27, and the back-side region 21 is constituted by the fourth region 29 through the sixth region 33.

The absorbent core 9 comprises a narrow part 35 which is arranged in the crotch region 19, and specifically, which is arranged across the first region 23 through the third region 27, and which has a short length in the width direction W.

The absorbent core 9 comprises a plurality of crotch region main grooves 41 in the narrow part 35 of the crotch region 19 (the second region 25 and the third region 27) which extend along the longitudinal direction L and which are spaced apart in the width direction W. Each of the plurality of crotch region main grooves 41 includes a plurality of crotch region sub-grooves 43 which extend in a direction along the width direction W.

Likewise, the absorbent core 9 comprises a plurality of abdomen-side region main grooves 45 in the abdomen-side region 17 (first region 23) which extend along the longitudinal direction L and which are spaced apart in the width direction W. Each of the plurality of abdomen-side region main grooves 45 includes a plurality of abdomen-side region sub-grooves 47 which extend along the width direction W.

Some of the plurality of crotch region main grooves 41 are connected to the abdomen-side region main grooves 45.

Likewise, the absorbent core 9 comprises a plurality of back-side region main grooves 49 in the back-side region 21 (the fourth region 29 through the sixth region 33) which extend along the longitudinal direction L and which are spaced apart in the width direction W. Each of the plurality of back-side region main grooves 49 includes a plurality of back-side region sub-grooves 51 which extend along the width direction W.

Some of the plurality of crotch region main grooves 41 are connected to the back-side region main grooves 49.

The absorbent core 9 comprises a first slit part 53 arranged in the abdomen-side region 17 through the crotch region 19 (specifically, the first region 23 and the second region 25) and a second slit part 55 arranged in the back-side region 21 (specifically, the fourth region 29 and the fifth region 31).

Though not illustrated, in the absorbent core 9, the average basis weight of the super absorbent polymer particles in the crotch region 19 (the second region 25 and the third region 27) is higher than the average basis weight of the super absorbent polymer particles in the entirety of the absorbent core 9 (the first region 23 through the sixth region 33). As a result, the absorbent core 9 has an excellent fit when worn (initial and over time), and the crotch region 19 has high liquid retention, whereby absorbency is less likely to be reduced even when liquid is repeatedly absorbed.

Though not illustrated, in the absorbent core 9, the ratio of super absorbent polymer particles in the crotch region 19 (the second region 25 and the third region 27) is higher than the ratio of super absorbent polymer particles in the entirety of the absorbent core 9 (the first region 23 through the sixth region 33). As a result, the integrally molded absorbent core 9 has an excellent fit when worn, there is little reduction in the water storage capacity of the plurality of crotch region main grooves 41, the liquid retention capacity of the crotch region 19 is high, whereby absorbency is less likely to be reduced even when the absorbent core 9 repeatedly absorbs liquid.

Though not illustrated, in the absorbent core 9, the average basis weight of the super absorbent polymer particles in the abdomen-side region 17 (the first region 23) is lower than the average basis weight of the super absorbent polymer particles in the entirety of the absorbent core 9 (the first region 23 through the sixth region 33). As a result, even if the absorbent core 9 repeatedly absorbs liquid, the thickness of the absorbent core 9 in the abdomen-side region 17 is unlikely to increase.

Though not illustrated, the average basis weight of the super absorbent polymer particles in the back-side region 21 (the fourth region 29 through the sixth region 33) is lower than the average basis weight of the super absorbent polymer particles in entirety of the absorbent core 9 (the first region 23 through the sixth region 33). As a result, even if the absorbent core 9 repeatedly absorbs liquid, the thickness of absorbent core 9 in back-side region 21 is unlikely to increase.

The absorbent core 9 is composed of a first layer 61 constituting a skin-facing surface 11 (the surface on the liquid permeable sheet 3 side) and a second layer 63 constituting a non-skin-facing surface 13 (the surface on the liquid impermeable sheet 5 side). The first layer 61 and the second layer 63 are adjacent to each other in the thickness direction T. Each of the first layer 61 and the second layer 63 contains the super absorbent polymer particles 37 and the pulp fibers 39.

The first layer 61 comprises a plurality of through hole parts 65 arranged in the crotch region 19 (the second region 25 and the third region 27) and a plurality of base parts 67. The through hole parts 65 extend along the longitudinal direction L and penetrate the first layer 61 in the thickness direction T. The base parts 67 are arranged between the plurality of through hole parts 65.

The second layer 63 includes a plurality of parts 69 corresponding to the through hole parts and a plurality of parts 71 corresponding to the base parts, respectively, at positions overlapping the plurality of through hole parts 65 and the base parts 67 in the thickness direction T.

The average density of the super absorbent polymer particles 37 in the base parts 67 constituting the skin-facing surface 11 is higher than the average density of the super absorbent polymer particles 37 in the parts 69 corresponding to the through hole parts. As a result, the base parts 67 have excellent liquid retention.

Furthermore, the average density of the super absorbent polymer particles 37 in the base parts 67 constituting the skin-facing surface 11 is higher than the average density of the super absorbent polymer particles 37 in the parts 71 corresponding to the base parts constituting the non-skin-facing surface 13. As a result, the parts 71 corresponding to the base parts can diffuse liquid in the longitudinal direction L, and liquid can more easily be transferred to the base parts 67, which have excellent liquid retention.

Since the crotch region main grooves 41 each have a plurality of crotch region sub-grooves 43 in the absorbent core 9, the volume and surface area of the entirety of the crotch region grooves including the crotch region main grooves 41 is increased, whereby it is easier to diffuse liquid in the longitudinal direction L and the width direction W.

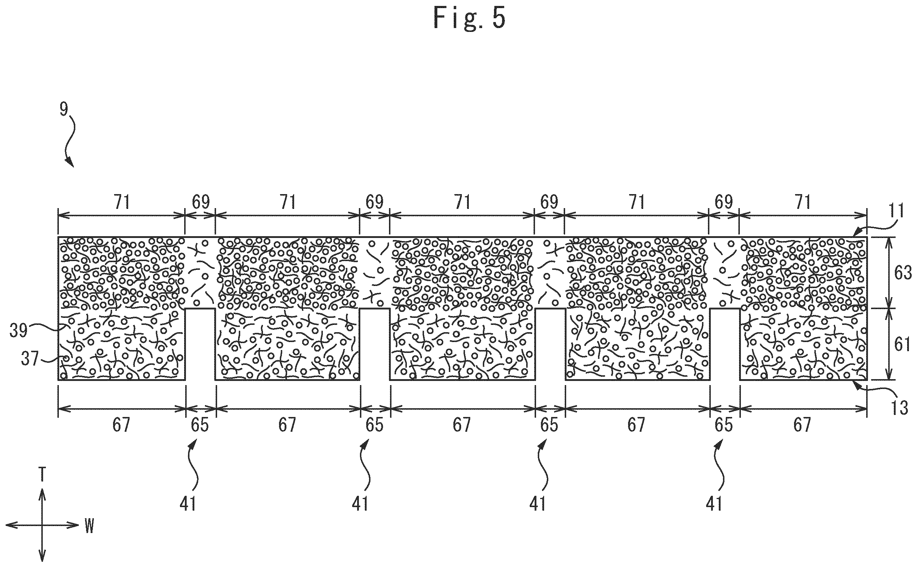

FIG. 5 is a view detailing an absorbent article, specifically, a disposable diaper (not illustrated), comprising an absorbent body (not illustrated) for an absorbent article, according to another embodiment of the present disclosure (hereinafter, sometimes referred to as the "second embodiment"). Specifically, FIG. 5 is an end view of an absorbent core 9 constituting the absorbent body according to the second embodiment, corresponding to the IV-IV end face of FIG. 3. Note that since the absorbent body according to the second embodiment is identical to the absorbent body 7 according to the first embodiment, the following description will focus on the differences.

The absorbent core 9 according to the second embodiment is composed of the first layer 61 constituting the non-skin-facing surface 13 (the surface on the liquid impermeable sheet 5 side) and the second layer 63 constituting the skin-facing surface 11 (the surface on the liquid permeable sheet 3 side). The first layer 61 and the second layer 63 are adjacent to each other in the thickness direction T. Each of the first layer 61 and the second layer 63 contains the super absorbent polymer particles 37 and the pulp fibers 39.

The average density of the super absorbent polymer particles 37 in the parts 71 corresponding to the base parts constituting the skin-facing surface 11 is higher than the average density of the super absorbent polymer particles 37 in the parts 69 corresponding to the through hole parts. As a result, the parts 71 corresponding to the base parts have excellent liquid retention.

Furthermore, the average density of the super absorbent polymer particles 37 in the parts 71 corresponding to the base parts constituting the skin-facing surface 11 is higher than the average density of the super absorbent polymer particles 37 in the base parts 67 constituting the non-skin-facing surface 13. As a result, the base parts 67 can diffuse liquid in the longitudinal direction L, and liquid can more easily be transferred to the parts 71 corresponding to the base parts, which have excellent liquid retention.

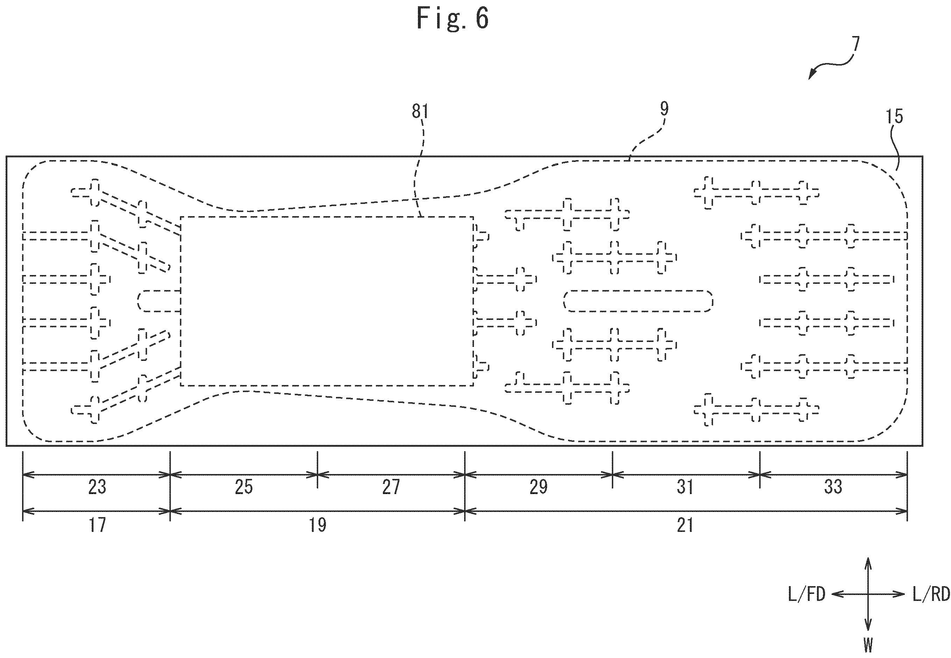

FIG. 6 is a view detailing an absorbent article, specifically, a disposable diaper (not illustrated), comprising an absorbent body 7 for an absorbent article, according to yet another embodiment of the present disclosure (hereinafter, sometimes referred to as the "third embodiment"). Specifically, FIG. 6 is a plan view of the absorbent body 7. Note that the absorbent body 7 according to the third embodiment is identical to the absorbent body 7 according to the first embodiment, and thus, the following description will focus on the differences.

The absorbent body 7 according to the third embodiment comprises, in this order, a core wrap 15 on the liquid permeable sheet side, an additional absorbent core 81, the absorbent core 9, and a core wrap 15 on the liquid impermeable sheet side, and the core wrap 15 on the liquid permeable sheet side, the absorbent core 9, and the core wrap 15 on the liquid impermeable sheet side are identical to those illustrated in the first embodiment.

The additional absorbent core 81, which is arranged between the core wrap 15 on the liquid permeable sheet side and the absorbent core 9, is arranged across a range which generally overlaps the absorbent core 9 in the crotch region 19, the additional absorbent core 81 is composed of pulp fibers, and the average basis weight of the additional absorbent core 81 is lower than the average basis weight of the absorbent core 9.

As a result, the crotch region main grooves (not illustrated) are reinforced while retaining the flexibility thereof, and rupture of the absorbent core 9 that starts at the crotch region main grooves can be prevented. The additional absorbent core 81 absorbs the liquid partially remaining in the crotch region main grooves, causing the super absorbent polymer particles to partially swell, preventing the additional absorbent core 81 from partially rupturing. Furthermore, since the pulp fibers in the absorbent core 9 become less bulky when wet as compared to when dry, though the wearer may feel discomfort due to swelling of the super absorbent polymer particles in the crotch region 19 after absorbing liquid, the additional absorbent core 81 can prevent this discomfort.

An example of a method of producing the absorbent core 9 according to the first embodiment will be described.

In the present description, the "conveying direction of the material or product" is referred to as the "MD direction", the "direction perpendicular to the MD direction on a horizontal plane" (i.e., the width direction of the production line) is referred to as the "CD direction", and the "direction perpendicular to these MD and CD directions" (i.e., the vertical direction of the production line) is referred to as the "TD direction." The rotation direction of suction drum 205 is referred to as the RT direction.

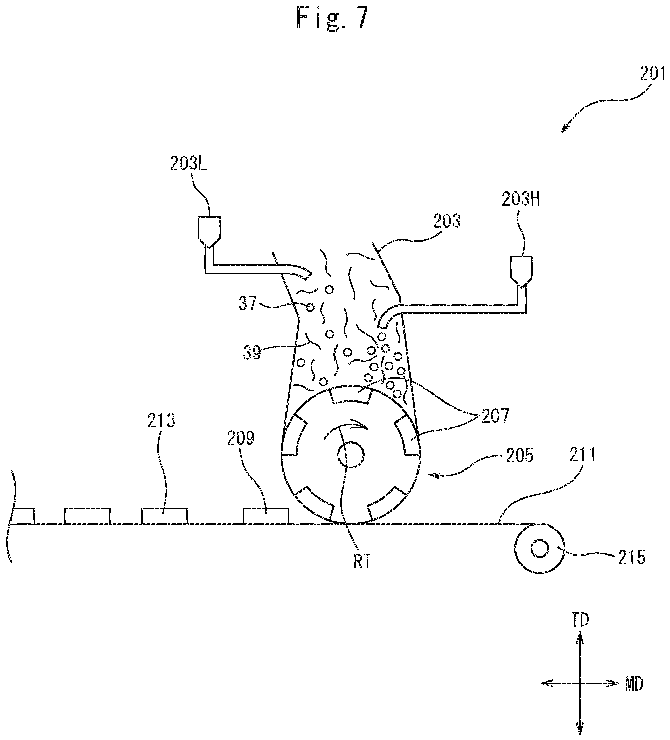

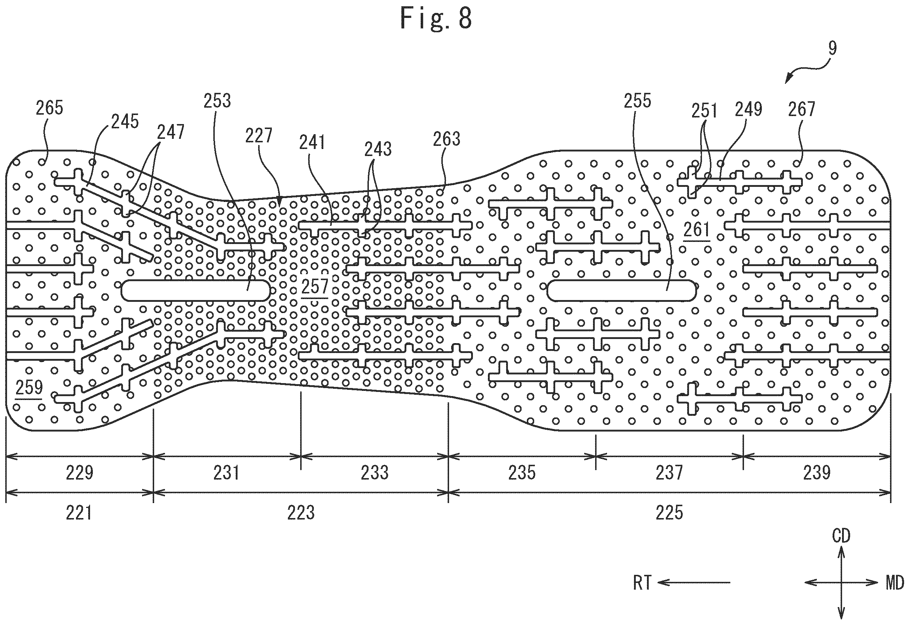

FIG. 7 is a schematic view of a production device 201 which is capable of producing the absorbent core 9 according to the first embodiment. FIG. 8 is a plan view of a mold member 207 provided on the outer circumferential surface of a suction drum 205 of the production device 201.

The production device 201 comprises a conveying pipe 203 for conveying absorbent material including opened pulp fibers 39 and the super absorbent polymer particles 37, and a rotatable suction drum 205 for suctioning the absorbent material discharged from conveying pipe nozzles 203L and 203H of the conveying pipe 203 and laminating the absorbent material on a plurality of concave mold members 207 arranged at regular intervals along the circumferential direction on the outer circumferential surface thereof to form a first stack 209, which will serve as the absorbent core 9 of the absorbent body 7 in subsequent processes.

The conveying pipe 203 includes the conveying pipe nozzle 203L, which discharges the super absorbent polymer particles 37 at a relatively low discharge ratio, and the conveying pipe nozzle 203H, which discharges the super absorbent polymer particles 37 at a relatively high discharge ratio. In the present embodiment, the mass ratio of the super absorbent polymer particles 37 contained in the conveying pipe nozzle 203L and the conveying pipe nozzle 203H is 30:70, respectively.

The production device 201 also includes an unwinding roller 215 for a core wrap continuous body 211, which places the first stack 209 on the outer peripheral surface of the suction drum 205, unwinds the long core wrap continuous body 211, which covers the first stack 209, and forms a second stack 213 by covering the first stack 209 with the core wrap continuous body 211.

The mold member 207 includes an abdomen-side part 221, a crotch part 223, a back-side part 225, and a narrowed part 227, which respectively correspond to the abdomen-side region 17, the crotch region 19, the back-side region 21, and the narrow part 35 of the absorbent core 9. The mold member 207 is configured such that when the mold member 207 is divided into six equal parts in the MD direction, from the front, into a first part 229, a second part 231, a third part 233, a fourth part 235, a fifth part 237, and a sixth part 239, the abdomen-side part 221 is constituted by the first part 229, the crotch part 223 is constituted by the second part 231 and the third part 233, and the back-side part 225 is constituted by the fourth part 235 through the sixth part 239. The abdomen-side part 221, the crotch part 223, and the back-side part 225 of the mold member 207 are recessed inward. The first part 229, the second part 231, the third part 233, the fourth part 235, the fifth part 237, and the sixth part 239 of the mold member 207 respectively correspond to the first region 23, the second region 25, the third region 27, the fourth region 29, the fifth region 31, and the sixth region 33 of the absorbent core 9.

The mold member 207 comprises a plurality of crotch part main ridges 241 and a plurality of crotch part sub-ridges 243 which respectively correspond to the plurality of crotch region main grooves 41 and the plurality of crotch region sub-grooves 43 of the absorbent core 9. The mold member 207 also comprises a plurality of abdomen-side part main ridges 245, a plurality of abdomen-side part sub-ridges 247, a plurality of back-side part main ridges 249, and a plurality of back-side part sub-ridges 251 which respectively correspond to the plurality of abdomen-side region main grooves 45, the plurality of abdomen-side region sub-grooves 47, the plurality of back-side region main grooves 49, and the plurality of back-side region sub-grooves 51 of the absorbent core 9. The mold member 207 also comprises a first slit part ridge 253 and a second slit part ridge 255 which respectively correspond to the first slit part 53 and the second slit part 55 of the absorbent core 9.

In order to change the length of the width direction W of the plurality of crotch region main grooves 41, the plurality of abdomen-side region main grooves 45, and the plurality of back-side region main grooves 49 of the absorbent core 9, it is sufficient to change the lengths of the plurality of crotch part main ridges 241, the plurality of abdomen-side part main ridges 245, and the plurality of back-side part main ridges 249 in the CD direction.

For example, the length of the plurality of crotch part main ridges 241 in the CD direction may be increased in order to shorten the length of the width direction W of the plurality of crotch region main grooves 41.

The plurality of crotch part main ridges 241, the plurality of crotch part sub-ridges 243, the plurality of abdomen-side part main ridges 245, the plurality of abdomen-side part sub-ridges 247, the plurality of back-side part main ridges 249, and the plurality of back-side part sub-ridges 251, as well as the first slit part ridge 253 and the second slit part ridge 255 protrude toward the near side.

The mold member 207 comprises a plurality of crotch part openings 263 in the crotch part body 257 excluding the plurality of crotch part main ridges 241, the plurality of crotch part sub-ridges 243, and the first slit part ridge 253 in the crotch part 223 (the second part 231 and the third part 233). The mold member 207 also comprises a plurality of abdomen-side part openings 265 in the abdomen-side part body 259 excluding the plurality of abdomen-side part main ridges 245, the plurality of abdomen-side part sub-ridges 247, and the first slit part ridge 253 in the abdomen-side part 221 (the first part 229). Further, in the back-side part 225 (the fourth part 235 through the sixth part 239), the mold member 207 comprises a plurality of back-side part openings 267 in a back-side part body 261 excluding the plurality of back-side part main ridges 249, the plurality of back-side part sub-ridges 251, and the second slit part ridge 255.

Each of the plurality of crotch part openings 263, the plurality of abdomen-side part openings 265, and the plurality of back-side part openings 267 has a circular shape and is identical in shape.

Furthermore, the plurality of crotch part openings 263, the plurality of abdomen-side part openings 265, and the plurality of back-side part openings 267 are arranged in a staggered manner. Note that though the plurality of crotch part openings 263, the plurality of abdomen-side part openings 265, and the plurality of back-side part openings 267 are illustrated in FIG. 8, this is to facilitate understanding thereof, and does not reflect the actual size, the actual opening ratio, etc.

The opening ratio of the plurality of crotch part openings 263 in the crotch part 223 is higher than the opening ratio of the plurality of abdomen-side part openings 265 in the abdomen-side part 221, and is higher than the opening ratio of the plurality of back-side part openings in the back-side part 225. As a result, the absorbent core 9, in which the basis weight of the super absorbent polymer particles in the crotch region 19 is higher than the average basis weight of the super absorbent polymer particles in the entirety of the absorbent core 9, can appropriately be formed by integral molding.

Note that the average density of the super absorbent polymer particles 37 in the base parts 67 constituting the skin-facing surface 11 of the absorbent core 9 being higher than the average density of the super absorbent polymer particles 37 in the parts 69 corresponding to the through hole parts, and the average density of the super absorbent polymer particles 37 in the base parts 67 constituting the skin-facing surface 11 being higher than the average density of the super absorbent polymer particles 37 in the parts 71 corresponding to the base parts constituting the non-skin-facing surface 13 can be produced by the methods described in Patent Literature 1, Patent Literature 2, Japanese Unexamined Patent Publication (Kokai) No. 2019-118722, etc., and thus, description thereof has been omitted.

In the absorbent body of the present disclosure, the absorbent core contains pulp fibers and super absorbent polymer particles and is integrally molded. The absorbent body and the absorbent core of the present disclosure have mutually perpendicular longitudinal, width, and thickness directions.

As the super absorbent polymer particles contained in the absorbent core according to the present disclosure, those known in the art can be used without particular limitation, and examples of the super absorbent polymer particles include polyacrylate-based, polysulfonatebased, maleic anhydride-based, polyacrylamide-based, polyvinyl alcohol-based, polyethylene oxide-based, polyaspartate-based, polyglutamate-based, polyalginate-based, starch-based, and cellulose-based super absorbent polymer particles; starch-based or cellulose-based super absorbent polymer particles such as starch-acrylic acid (salt) graft copolymers, saponified starchacrylonitrile copolymers, crosslinked products of sodium carboxymethyl cellulose, etc.

The absorbent core according to the present disclosure comprises pulp fibers in addition to the super absorbent polymer particles.

The absorbent core according to the present disclosure may further comprise other materials known in the art, such as fibers other than the pulp fibers, including natural fibers, synthetic fibers, and semi-synthetic fibers. Examples of natural fibers include regenerated cellulose fibers. Examples of regenerated cellulose fibers include rayon fibers, such as viscose rayon, polynosic, and modal obtained from viscose, cuprammonium rayon fibers (also referred to as "cupra") obtained from a cuprammonium salt solution of cellulose, and lyocell and tencel obtained by an organic solvent spinning method using an organic solvent, which is a mixed solution of an organic compound and water, without going through a cellulose derivative.

Examples of semi-synthetic fibers include semi-synthetic cellulose fibers, such as acetate fibers, for example, triacetate fibers and diacetate fibers.

The absorbent body of the present disclosure may further comprise a core wrap which covers the skin-facing surface and/or the non-skin-facing surface of the absorbent core. The core wrap may be any known in the art, such as tissue, a nonwoven fabric (for example, an air-through nonwoven fabric), etc.

The absorbent core according to the present disclosure is partitioned in the longitudinal direction into an abdomen-side region, a crotch region, and a back-side region, in order from the front, and comprises a narrow part which is arranged in the crotch region and has a short length in the width direction.

The narrow part can be arranged over a part or the entire surface of the crotch region, and may be arranged extending to the abdomen-side part and/or the back-side part.

The absorbent core according to the present disclosure is partitioned into a crotch region arranged at the crotch of the wearer, an abdomen-side region in front of the crotch region, and a back-side region at the rear of the crotch region, depending on the absorbent article.

The absorbent core can be partitioned into six equal parts, for example, a first region through a sixth region, in order from the front, wherein the abdomen-side region is constituted by the first region, the crotch region is constituted by the second region and the third region, and the back-side region is constituted by the fourth region through the sixth region.

The absorbent core according to the present disclosure comprises a plurality of crotch region grooves (crotch region main grooves) in the narrow part of the crotch region which extend along the longitudinal direction and which are spaced apart in the width direction, which facilitates the diffusion of liquid in the longitudinal direction.

The plurality of crotch region grooves (crotch region main grooves) may either lack a bottom (through holes) or include a bottom.

It is preferable that each crotch region groove (crotch region main groove) be independent and not be connected to other crotch region grooves (crotch region main grooves) in order to diffuse liquid in the longitudinal direction.

In the absorbent core according to the present disclosure, at least a part of the plurality of crotch region grooves (crotch region main grooves) can have a plurality of crotch region sub-grooves extending in a direction along the width direction, which increases the volume and surface area of the crotch region grooves, making it easier to diffuse liquid in the longitudinal and width directions.

Each of the plurality of crotch region sub-grooves preferably has a terminal end at an end opposite to the end connected to the crotch region main groove in order to diffuse liquid in the longitudinal direction and move it into the inside of the absorbent core.

The absorbent core according to the present disclosure can comprise one or more abdomen-side region grooves (abdomen-side region main grooves) extending along the longitudinal direction in the abdomen-side region. In this case, each of the plurality of crotch region grooves (crotch region main grooves) may be connected to the abdomen-side region groove (abdomen-side region main groove). The length of each of the plurality of crotch region grooves (crotch region main grooves) in the width direction may be the same as, shorter than, or longer than, and preferably shorter than, the length of the abdomen-side region groove (abdomen-side region main groove) in the width direction. As a result, the absolute amount of the super absorbent polymer particles in the narrow part of the crotch region can be more easily secured.

The absorbent core according to the present disclosure can comprise one or more back-side region grooves (back-side region main grooves) in the back-side region which extend along the longitudinal direction. In this case, each of the plurality of crotch region grooves (crotch region main grooves) may be connected to a back-side region groove (back-side region main groove). Furthermore, the length of each of the plurality of crotch region grooves (crotch region main grooves) in the width direction may be the same as, shorter than, or longer than, and preferably shorter than, the length of the back-side region groove (back-side region main groove) in the width direction. As a result, the absolute amount of the super absorbent polymer particles in the narrow part of the crotch region can be more easily secured.

When the absorbent core according to the present disclosure comprises one or more abdomen-side region grooves (abdomen-side region main grooves) in the abdomen-side region, at least a part of the one or more abdomen-side region main grooves can comprise a plurality of abdomen-side region sub-grooves which extend in a direction along the width direction. As a result, liquid can more easily be diffused in the longitudinal direction and moved in the width direction in the abdomen-side region.

When the absorbent core according to the present disclosure comprises one or more back-side region grooves (back-side region main grooves) in the back-side region, at least a part of the one or more back-side region main grooves can comprise a plurality of back-side region sub-grooves extending in the width direction. As a result, liquid can more easily be diffused in the longitudinal direction and moved in the width direction in the back-side region.

In the absorbent core according to the present disclosure, at least a part of the plurality of crotch region grooves (crotch region main grooves) may be connected to the abdomen-side region grooves (abdomen-side region main grooves) and may be connected to the back-side region grooves (back-side region main grooves). As a result, liquid which reaches the crotch region of the absorbent core can quickly be transferred to the abdomen-side region and the back-side region.

In the absorbent core according to the present disclosure, the average basis weight of the super absorbent polymer particles in the crotch region is higher than the average basis weight of the super absorbent polymer particles in the entirety of the absorbent core, and the average basis weight of the super absorbent polymer particles in the crotch region is preferably 5.0% or more, more preferably 7.0% or more, and further preferably 9.0% or more higher than the average basis weight of the super absorbent polymer particles in the entirety of the absorbent core. Further, the average basis weight of the super absorbent polymer particles in the crotch region is preferably 20.0% or less, more preferably 17.0% or less, and further preferably 15.0% or less high than the average basis weight of the super absorbent polymer particles in the entirety of the absorbent core. As a result, the absorbent core has excellent fit when worn (initial and over time), and the crotch region has high liquid retention, whereby absorbency is less likely to be reduced even when liquid is repeatedly absorbed.

The absorbent core according to the present disclosure can preferably have an average basis weight of 100 to 1,000 g/m$^2$, more preferably 200 to 800 g/m$^2$, and further preferably 250 to 650 g/m$^2$.

The absorbent core according to the present disclosure can preferably have an average basis weight of the super absorbent polymer particles of 50 to 800 g/m$^2$, more preferably 100 to 650 g/m$^2$, and further preferably 125 to 500 g/m$^2$.

Furthermore, in the absorbent core according to the present disclosure, the average basis weight of the super absorbent polymer particles in the abdomen-side region is preferably lower than the average basis weight of the super absorbent polymer particles in the entirety of the absorbent core, and the average basis weight of the super absorbent polymer particles in the abdomen-side region is more preferably 1.0 to 10.0 mass%, further preferably 1.5 to 7.0 mass%, and even further preferably 2.0 to 5.0 mass% lower than the average basis weight of the super absorbent polymer particles in the entirety of the absorbent core. As a result, even if the absorbent core repeatedly absorbs liquid, the thickness of the absorbent core in the abdomen-side region is unlikely to increase, and the absorbent article comprising the absorbent core has excellent wearing comfort in the portion (abdomen-side portion) corresponding to the abdomen-side region of the absorbent core.

Furthermore, in the absorbent core according to the present disclosure, the average basis weight of the super absorbent polymer particles in the back-side region is preferably lower than the average basis weight of the super absorbent polymer particles in the entirety of the absorbent core, and the average basis weight of the super absorbent polymer particles in the back-side region is more preferably 1.0 to 10.0 mass%, further preferably 2.0 to 8.0 mass%, and even further preferably 3.0 to 7.0 mass% lower than the average basis weight of the super absorbent polymer particles in the entirety of the absorbent core. As a result, even if the absorbent core repeatedly absorbs liquid, the thickness of the absorbent core in the back-side region is unlikely to increase, and the absorbent article comprising the absorbent core has excellent wearing comfort in the portion (back-side portion) corresponding to the back-side region of the absorbent core.

In the absorbent core according to the present disclosure, the ratio of the super absorbent polymer particles in the crotch region is higher than that in the back-side region, and the ratio of the super absorbent polymer particles in the crotch region is preferably higher than that in the back-side region by 2.0% or more, more preferably 3.0% or more, and further preferably 4.0% or more. The ratio of the super absorbent polymer particles in the crotch region is preferably higher than that in the back-side region by 10.0% or less, more preferably 9.0% or less, and further preferably 8.0% or less. As a result, the absorbent body has an excellent fit when worn, the water storage capacity of the plurality of crotch region grooves is less likely to be reduced, the liquid retention amount of the crotch region is high, whereby absorbency is less likely to be reduced even when liquid is repeatedly absorbed.

In the absorbent core according to the present disclosure, the ratio of the super absorbent polymer particles in the crotch region is higher than that in the abdomen-side region, and the ratio of the super absorbent polymer particles in the crotch region is preferably 0.5% or more, more preferably 1.0% or more, and further preferably 1.5% or more higher than that in the abdomen-side region. The ratio of the super absorbent polymer particles in the crotch region is preferably 10.0% or less, more preferably 9.0% or less, and further preferably 8.0% or less higher than that in the abdomen-side region. As a result, the absorbent body has an excellent fit when worn, the water storage capacity of the plurality of crotch region grooves is less likely to be reduced, the liquid retention amount of the crotch region is high, whereby absorbency is less likely to be reduced even when liquid is repeatedly absorbed.

In the present description, the average basis weight of the super absorbent polymer particles and pulp fibers in predetermined regions of the absorbent core, and the ratio (mass ratio) of the super absorbent polymer particles can be measured as follows.

[Creation of Calibration Curve]

(1) Super absorbent polymer particles are collected from the absorbent core.

(2) The collected super absorbent polymer particles are immersed in 200 mL of 1% KCl aqueous solution at varying amounts, stirred for 3 minutes, and the supernatant is filtered through filter paper (ADVANTEC filter paper: grade 2), and the salt concentration of the filtrate is measured with a sodium ion meter (manufactured by HORIBA, Ltd., compact water quality meter LAQUAtwin NA-11).

(3) A calibration curve between the mass of super absorbent polymer particles and the sodium salt concentration is created.

[Measurement of Average Basis Weight and Ratio]

(1) The absorbent article is stretched on a flat surface until it is free of wrinkles, and a desired region of the absorbent core is cut from the absorbent article to form a small measurement sample. Next, the mass MAA (g) and area S (m$^2$) of the small measurement sample are measured.

(2) The small measurement sample is immersed in 200 mL of a 1% KCL aqueous solution, stirred for 3 minutes, and the supernatant is filtered to measure the sodium salt concentration of the small measurement sample. Using the calibration curve, the mass of the super absorbent polymer particles: $M_{SAP}$ (g) is calculated from the sodium salt concentration, and the average basis weight of the super absorbent polymer particles: $BW_{SAP}$ (g/m$^2$) is calculated using the following formula:

$$BW_{SAP} = M_{SAP}/S$$

(3) The small measurement sample is washed with a sufficient amount of ion-exchanged water to remove the sodium salt concentration measurement residue, and the pulp fibers and super absorbent polymer particles are suspended and removed. The remaining member is dried in a hot air dryer at 105°C, and the mass: $M_{MEM}$ (g) of the member is then measured.

(4) The mass of the absorbent core of the small measurement sample: $M_{AC}$ (g) is calculated using the following formula:

$$M_{AC}=M_{AA}-M_{MEM}$$

(5) The average basis weight of the absorbent core in a predetermined region: $BW_{AC}$ (g/m$^2$) is calculated using the following formula:

$$BW_{AC}=M_{AC}/S$$

(6) The average basis weight of the pulp fibers in a predetermined region: $BW_{PF}$ (g/m$^2$) is calculated using the following formula:

$$BW_{PF}=BW_{AC}-BW_{SAP}$$

(7) The ratio of the super absorbent polymer particles: R (mass%) is calculated using the following formula:

$$R=100\times BW_{SAP}/BW_{AC}$$

As another method, in the present description, the average basis weight of the super absorbent polymer particles and the pulp fibers in predetermined regions of the absorbent core can be calculated by separating the super absorbent polymer particles and the pulp fibers from the predetermined regions of the absorbent core.

The absorbent body of the present disclosure can comprise an additional absorbent core on the skin-facing surface side or non-skin-facing surface side of the absorbent core. The additional absorbent core can be arranged in a region overlapping the abdomen-side region, the crotch region, and/or the back-side region of the absorbent core in the thickness direction, and/or in a region overlapping the narrow part in the thickness direction, but is preferably arranged in a region overlapping the crotch region and the narrow part of the absorbent core in the thickness direction. Furthermore, the average basis weight of the additional absorbent core is preferably lower than the average basis weight of the absorbent core overlapping in the thickness direction.

As a result, the crotch region grooves can be reinforced while maintaining the flexibility of the crotch region grooves, and rupture of the absorbent core that starts at the crotch region grooves can be prevented. Furthermore, liquid remaining partially in the crotch region grooves is absorbed by the additional absorbent core, causing the super absorbent polymer particles to partially swell, preventing the additional absorbent core from rupturing partially.

The additional absorbent core can be a layer containing the pulp fibers, a layer composed of the pulp fibers, a layer containing the super absorbent polymer particles, a layer composed of the super absorbent polymer particles, etc., and is preferably a layer composed of the pulp fibers. Since the volume of the pulp fibers in the absorbent core is reduced from the dry state when wet, after absorbing liquid, the wearer may feel discomfort due to swelling of the super absorbent polymer particles in the crotch region, but the additional absorbent core, which is a layer composed of the pulp fibers, can prevent this discomfort.

The absorbent core according to the present disclosure can comprise a skin-facing surface and a non-skin-facing surface, and can comprise a first layer and a second layer which are adjacent to each other in the thickness direction.

As long as the first layer and second layer are adjacent to each other in the thickness direction of the absorbent article, the first layer and second layer can be arranged on the skin-facing and non-skin-facing sides or the non-skin-facing and skin-facing sides of the absorbent core, respectively.

The first layer has a plurality of through hole parts which are arranged in a crotch region and which correspond to the plurality of crotch region grooves, and base parts which are arranged between the plurality of through hole parts, and the second layer has a plurality of parts corresponding to the through hole parts and parts corresponding to the base parts, respectively, at positions overlapping the plurality of through hole parts and the base parts in the thickness direction.

It is preferable that the average density of the super absorbent polymer particles in the base parts or the parts corresponding to the base parts constituting the skin-facing surface be higher than the average density of the super absorbent polymer particles in the parts corresponding to the through hole parts. As a result, the absorbent body has high liquid retention in the crotch region, and absorbency is less likely to be reduced even when the absorbent body repeatedly absorbs liquid.

Furthermore, it is preferable that the average density of the super absorbent polymer particles in the base parts or the parts corresponding to the base parts constituting the skin-facing surface be higher than the average density of the super absorbent polymer particles in the base parts or the parts corresponding to the base parts constituting the non-skin-facing surface. As a result, the absorbent body has high liquid retention in the crotch region, and absorbency is less likely to be reduced even when the absorbent body repeatedly absorbs liquid.

In the absorbent core according to the present disclosure, the first layer preferably contains the super absorbent polymer particles at an average basis weight of 30 to 100 g/m$^2$, and more preferably 40 to 60 g/m$^2$, and the second layer preferably contains the super absorbent polymer particles at an average basis weight of 100 to 180 $g/m^2$, and more preferably 120 to 160 $g/m^2$. The first layer and the second layer preferably respectively contain 20 to 45% by weight and 55 to 80% by weight, and more preferably 25 to 35% by weight and 65 to 75% by weight, of the super absorbent polymer particles relative to the total weight of the super absorbent polymer particles contained in the first layer and the second layer.

In the absorbent core according to the present disclosure, each of the first layer and the second layer contains the pulp fibers, preferably at an average basis weight of 50 to 150 $g/m^2$, and more preferably 80 to 100 $g/m^2$.

In the absorbent core according to the present disclosure, the thickness of each of the first layer and the second layer is preferably 0.1 to 5.0 mm, more preferably 0.5 to 4.0 mm, and further preferably 1.0 to 3.0 mm.

The thicknesses of the first layer and the second layer can be measured in a non-contact manner using a laser displacement meter (for example, a high-precision two-dimensional laser displacement meter LJ-G series (model: LJ-G030) manufactured by Keyence Corporation).

In the absorbent core according to the present disclosure, the average density of the super absorbent polymer particles in the base parts or the parts corresponding to the base parts constituting the skin-facing surface is higher, preferably 0.01 to 0.20 $g/cm^3$, and more preferably by 0.05 to 0.20 $g/cm^3$, higher than the average density of the super absorbent polymer particles contained in the parts corresponding to the through holes.

The average density of the super absorbent polymer particles in the base parts or the parts corresponding to the base parts constituting the skin-facing surface is preferably higher, more preferably 0.01 to 0.15 $g/cm^3$ higher, and further preferably 0.05 to 0.15 $g/cm^3$ higher, than the average density of the super absorbent polymer particles in the base parts or the parts corresponding to the base parts constituting the non-skin-facing surface, from the viewpoint of the effects of the present disclosure.

As used herein, the average density of super absorbent polymer particles is measured as follows.

(1) The absorbent article is cold sprayed, and the liquid permeable sheet, liquid impermeable sheet, and core wrap are peeled off to obtain the absorbent core.

(2) Five or more absorbent core samples of a predetermined area are cut from a predetermined region of the absorbent core so as to have a predetermined area (for example, 4 mm $\times$ 4 mm).

(3) An FS-60DS (measuring surface 44 mm (diameter), measuring pressure 3 $g/cm^2$) manufactured by DAIEI KAGAKU SEIKI MFG. CO., LTD. is prepared, and five different parts of the absorbent core (or absorbent body) are pressurized under standard conditions (temperature 23±2°C, relative humidity 50±5%). The thickness of each part ten seconds after pressurizing is measured and the average of the five measured values is used as the thickness of the absorbent core sample.

(4) The super absorbent polymer particles contained in each sample are sorted, and the total mass of the super absorbent polymer particles contained in each sample is measured. The measured value is divided by the sample volume, which is obtained from the sample thickness and sample area described below, to obtain the average density.

In the present description, the level of the average density of super absorbent polymer particles can be evaluated, for example, as follows.

After the absorbent article is immersed in liquid nitrogen and frozen, and the absorbent article is cut in the thickness direction using a razor or the like to obtain a cross-sectional sample at a plane intersecting the direction in which the grooves extend. The sample is then returned to room temperature, and a cross-sectional image at a magnification of 50-fold is obtained using an electron microscope (for example, Keyence VE7800). The level of the average density of the super absorbent polymer particles in the cross-sectional image is then visually evaluated.

[Examples]

The present disclosure will be described below with reference to Examples, but the present disclosure is not limited to these Examples.

[Production Example 1]

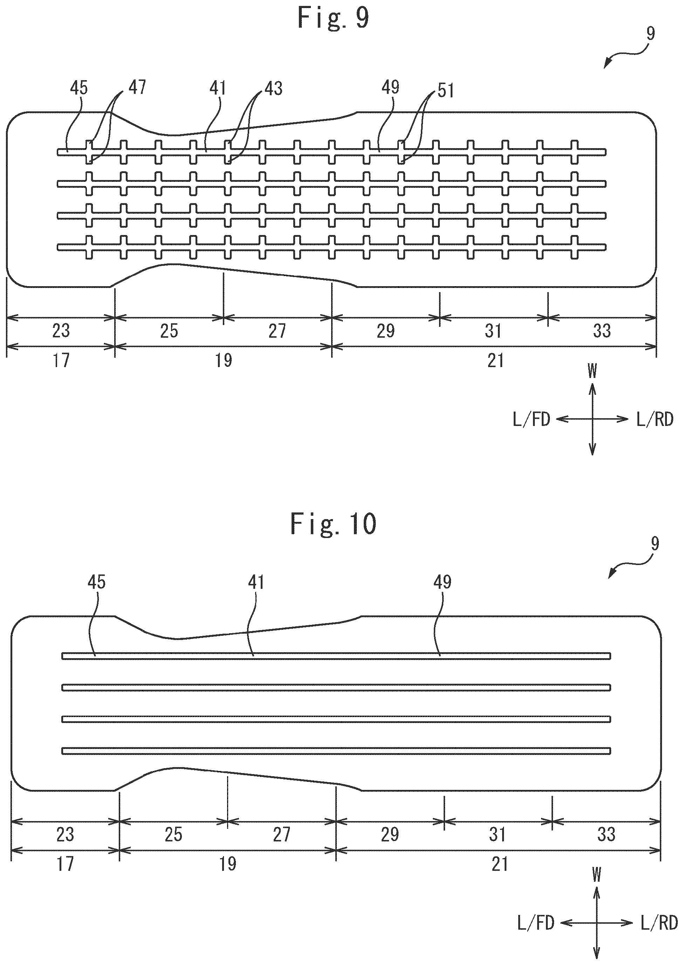

The absorbent core 9 (absorbent core No. 1) shown in FIG. 9 was produced by a method similar to that shown in FIGS. 7 and 8. The absorbent core No. 1 had four main grooves (crotch region main grooves 41 + abdomen-side region main grooves 45 + back-side region main grooves 49) extending in the longitudinal direction L across the abdomen-side region 17, the crotch region 19, and the back-side region 21, and each of the four main grooves had plurality of sub-grooves (crotch region sub-grooves 43 + abdomen-side region sub-grooves 47 + back-side region sub-grooves 51).

In the production device 201 shown in FIG. 7, the pulp fibers and the super absorbent polymer particles were sprinkled at a mass ratio of approximately 50:50.

In the mold member 207 for forming the absorbent core No. 1, the opening ratios of the abdomen-side part 221, the crotch part 223, and the back-side part 225 were 25%, 40% and 25%, respectively.

The average basis weight of the super absorbent polymer particles (SAP basis weight, $g/m^2$), the average basis weight of the pulp fibers (pulp basis weight, $g/m^2$), and the ratio of the super absorbent polymer particles (SAP ratio, mass%) were measured in the abdomen-side region, the crotch region, and the back-side region of absorbent core No. 1. The SAP basis weight of the entirety of the absorbent core was also measured. The results are shown in Table 1.

The absorbent core No. 1 was covered with two tissue sheets (basis weight: 16 $g/m^2$, 400 mm $\times$ 150 mm) as a core wrap with a hot melt adhesive interposed therebetween. The absorbent core No. 1 covered with the two tissue sheets was pressed with a hydraulic press to adjust the thickness of the absorbent core, whereby the absorbent body No. 1 was formed.

A liquid permeable sheet (air-through nonwoven fabric) was attached to the second layer side of absorbent body No. 1, and a liquid impermeable sheet (polyethylene film) was attached to the first layer side to produce a simple absorbent article No. 1.

[Production Example 2]

The absorbent core 9 (absorbent core No. 2), the absorbent body No. 2, and the absorbent article No. 2 shown in FIG. 10 were produced in the same manner as in Production Example 1. The absorbent core No. 2 had four main grooves (crotch region main grooves 41 + abdomen-side region main grooves 45 + back-side region main grooves 49) extending in the longitudinal direction L across the abdomen-side region 17, the crotch region 19, and the back-side region 21, and each of the four main grooves did not have sub-grooves.

The SAP basis weight, the pulp basis weight, and the SAP ratio were measured in the abdomen-side region, the crotch region, and the back-side region of the absorbent core No. 2. The SAP basis weight of the entirety of the absorbent core was also measured. The results are shown in Table 1.

[Reference Production Example 3]

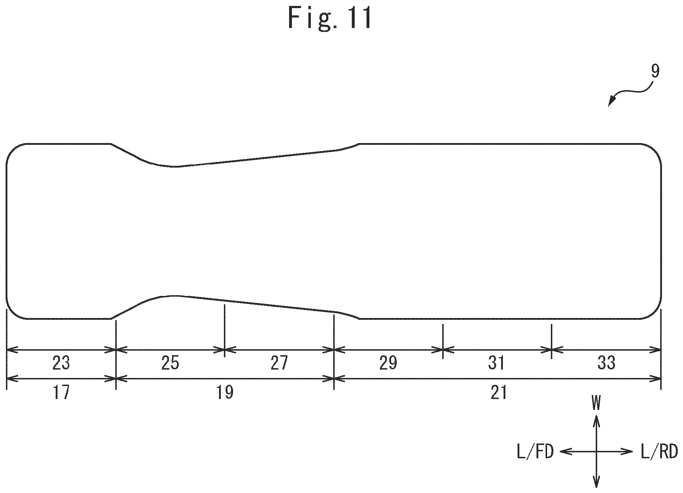

The absorbent core 9 (absorbent core No. 3), the absorbent body No. 3, and the absorbent article No. 3 shown in FIG. 11 were produced in the same manner as in Production Example 1. The absorbent core No. 3 did not have main grooves or sub-grooves.

The SAP basis weight, the pulp basis weight, and the SAP ratio were measured in the abdomen-side region, the crotch region, and the back-side region of absorbent core No. 3. The SAP basis weight of the entirety of the absorbent core was also measured. The results are shown in Table 1.

[Comparative Production Example 4]

The absorbent core No. 4, the absorbent body No. 4, and the absorbent article No. 4, were produced in the same manner as in Production Example 1, except that the opening ratio of each of the abdomen-side part 221, the crotch part 223, and the back-side part 225 was set to 40% (constant).

The SAP basis weight, the pulp basis weight, and the SAP ratio were measured in the abdomen-side region, the crotch region, and the back-side region of the absorbent core No. 4. The SAP basis weight of the entirety of the absorbent core was also measured. The results are shown in Table 1.

[Comparative Production Example 5]

The absorbent core No. 5, the absorbent body No. 5, and the absorbent article No. 5, were produced in the same manner as in Production Example 2, except that the opening ratio of each of the abdomen-side part 221, the crotch part 223, and the back-side part 225 was set to 40% (constant).

The SAP basis weight, the pulp basis weight, and the SAP ratio were measured in the abdomen-side region, the crotch region, and the back-side region of the absorbent core No. 5. The SAP basis weight of the entirety of the absorbent core was also measured. The results are shown in Table 1.

[Comparative Reference Production Example 6]

The absorbent core No. 6, the absorbent body No. 6, and the absorbent article No. 6, were produced in the same manner as in Reference Production Example 3, except that the opening ratio of each of the abdomen-side part 221, the crotch part 223, and the back-side part 225 was set to 40% (constant).

The SAP basis weight, the pulp basis weight, and the SAP ratio were measured in the abdomen-side region, the crotch region, and the back-side region of the absorbent core No. 6. The SAP basis weight of the entirety of the absorbent core was also measured. The results are shown in Table 1.

[Table 1]

Table 1

| Prod Ex No. | Absorbent core No. | | Abdomen-side region | Crotch region | | Back-side region | | | Entire core |
|---|---|---|---|---|---|---|---|---|---|
| | | | First region | Second region | Third region | Fourth region | Fifth region | Sixth region | |
| Prod Ex 1 | No. 1 | SAP basis weight | 183 | 202 | | 174 | | | 184 |
| | | Pulp basis weight | 150 | 124 | | 126 | | | |
| | | SAP ratio | 55.0% | 62.0% | | 58.0% | | | |
| Prod Ex 2 | No. 2 | SAP basis weight | 201 | 209 | | 168 | | | 185 |
| | | Pulp basis weight | 137 | 146 | | 138 | | | - |
| | | SAP ratio | 59.5% | 58.9% | | 54.9% | | | - |
| Ref Prod Ex 3 | No. 3 | SAP basis weight | 185 | 174 | | 139 | | | 177 |
| | | Pulp basis weight | 153 | 198 | | 170 | | | - |
| | | SAP ratio | 54.7% | 46.8% | | 45.0% | | | - |
| Comp Prod Ex 4 | No. 4 | SAP basis weight | 219 | 171 | | 171 | | | 184 |
| | | Pulp basis weight | 125 | 137 | | 140 | | | - |
| | | SAP ratio | 63.7% | 55.5% | | 55.0% | | | - |

(continued)

| Prod Ex No. | Absorbent core No. | | Abdomen-side region | Crotch region | | Back-side region | | | Entire core |
|---|---|---|---|---|---|---|---|---|---|
| | | | First region | Second region | Third region | Fourth region | Fifth region | Sixth region | |
| Comp Prod Ex 5 | No. 5 | SAP basis weight | 191 | 163 | | 160 | | | 185 |
| | | Pulp basis weight | 147 | 159 | | 149 | | | - |
| | | SAP ratio | 56.5% | 50.6% | | 51.8% | | | - |
| Comp Ref Prod Ex 6 | No. 6 | SAP basis weight | 177 | 159 | | 144 | | | 177 |
| | | Pulp basis weight | 171 | 179 | | 168 | | | - |
| | | SAP ratio | 50.9% | 47.0% | | 46.2% | | | - |

[Example 1 and Comparative Example 1]

The absorbent articles No. 1 and No. 4 were subjected to the absorbency test specified below to evaluate the absorption times thereof. The results are shown in Table 2.

[Absorbency Test]

(1-1) The absorbent article is set in a U-shaped device that is substantially U-shaped in a side view. The absorbent article is set in the U-shaped device so that the center position of the absorbent body is aligned with the center of the U-shaped device (the position with the lowest height) in the longitudinal direction.

<First Cycle>

(1-2) 120 mL of artificial urine (first injection) is injected from a burette into the center position of the absorbent body at a rate of 120 mL/12 seconds.
(1-3) The time from the start of the first injection of artificial urine to the time when the artificial urine in the U-shaped device is completely consumed is recorded as the absorption time (120 mL).
(1-4) Three minutes after the start of the first injection of artificial urine, the diffusion length (120 mL) of the artificial urine from the center position in the longitudinal direction of the absorbent body to the abdomen-side and back-side is recorded from both the liquid permeable sheet (TS) side and the liquid impermeable sheet (BS) side.

<Second Cycle>

(1-5) 10 minutes after the start of the first injection of artificial urine, 120 mL of artificial urine (second injection) is injected from a burette into the center position of the absorbent body at a rate of 120 mL/12 seconds.
(1-6) The time from the start of the second injection of artificial urine to the time when the artificial urine in the U-shaped device is completely consumed is recorded as the absorption time (240 mL).
(1-7) Three minutes after the start of the second injection of artificial urine, the diffusion length (240 mL) of the artificial urine from the center position in the longitudinal direction of the absorbent body to the abdomen-side and back-side is recorded from both the liquid permeable sheet (TS) side and the liquid impermeable sheet (BS) side.
The artificial urine was prepared by dissolving 200 g of urea, 80 g of sodium chloride, 8 g of magnesium sulfate, 3 g of calcium chloride, and approximately 1 g of dye (Blue No. 1) in 10 L of ion-exchanged water.

## EP 4 574 116 A1

[Table 2]

Table 2

| Example No. | Ex 1 | Comp Ex 1 |
|---|---|---|
| Absorbent article No. | No. 1 | No. 5 |
| Absorption time (120 mL)/s | 34 | 41 |
| Absorption time (240 mL)/s | 84 | 122 |
| Diffusion length of artificial urine on TS side (120 mL)/mm | 226 | 213 |
| Diffusion length of artificial urine on BS side (120 mL)/mm | 252 | 234 |
| Diffusion length of artificial urine on TS side (240 mL)/mm | 231 | 232 |
| Diffusion length of artificial urine on BS side (240 mL)/mm | 257 | 251 |

DESCRIPTION OF REFERENCE SIGNS

1 disposable diaper
7 absorbent body
9 absorbent core
15 core wrap
17 abdomen-side region
19 crotch region
21 back-side region
23 first region
25 second region
27 third region
29 fourth region
31 fifth region
33 sixth region
35 narrow part
37 super absorbent polymer particles
39 pulp fibers
41 crotch region main groove
43 crotch region sub-groove
61 first layer
63 second layer
65 through hole part
67 base part
69 part corresponding to through hole part
71 part corresponding to base part
L longitudinal direction
W width direction
T thickness direction

## Claims

1. An absorbent body for an absorbent article, comprising an integrally molded absorbent core, the absorbent core comprising pulp fibers and super absorbent polymer particles, and having mutually perpendicular longitudinal, width, and thickness directions, wherein

the absorbent core is partitioned in the longitudinal direction into an abdomen-side region, a crotch region, and a back-side region, in order from a front, and comprises a narrow part which is arranged in the crotch region and has a short length in the width direction,

the absorbent core comprises a plurality of crotch region grooves in the narrow part in the crotch region which extend along the longitudinal direction and which are spaced apart in the width direction, and

an average basis weight of the super absorbent polymer particles in the crotch region in the absorbent core is higher than an average basis weight of the super absorbent polymer particles in the entirety of the absorbent core.

2. The absorbent body according to claim 1, wherein in the absorbent core, a ratio of the super absorbent polymer particles in the crotch region is higher than a ratio of the super absorbent polymer particles in the back-side region.

3. The absorbent body according to claim 1 or 2, wherein in the absorbent core, a ratio of the super absorbent polymer particles in the crotch region is higher than a ratio of the super absorbent polymer particles in the abdomen-side region.

4. The absorbent body according to any one of claims 1 to 3, wherein the absorbent core comprises a skin-facing surface and a non-skin-facing surface, and also comprises a first layer and a second layer which are adjacent to each other in the thickness direction,

the first layer has a plurality of through hole parts which are arranged in the crotch region and which correspond to the plurality of crotch region grooves, and base parts which are arranged between the plurality of through hole parts,

the second layer has a plurality of parts corresponding to the through hole parts and parts corresponding to the base parts, respectively, at positions overlapping the plurality of through hole parts and the base parts in the thickness direction, and

in the base parts or the parts corresponding to the base parts constituting the skin-facing surface, an average density of the super absorbent polymer particles is higher than an average density of the super absorbent polymer particles in the parts corresponding to the through hole parts.

5. The absorbent body according to claim 4, wherein an average density of the super absorbent polymer particles in the base parts or the parts corresponding to the base parts constituting the skin-facing surface is higher than an average density of the super absorbent polymer particles in the base parts or the parts corresponding to the base parts constituting the non-skin-facing surface.

6. The absorbent body according to any one of claims 1 to 5, wherein in the absorbent core, an average basis weight of the super absorbent polymer particles in the abdomen-side region is lower than an average basis weight of the super absorbent polymer particles in the entirety of the absorbent core.

7. The absorbent body according to any one of claims 1 to 6, wherein in the absorbent core, an average basis weight of the super absorbent polymer particles in the back-side region is lower than an average basis weight of the super absorbent polymer particles in the entirety of the absorbent core.

8. The absorbent body according to any one of claims 1 to 7, wherein at least a part of the plurality of crotch region grooves further comprises a plurality of crotch region sub-grooves which extend in a direction along the width direction.

9. The absorbent body according to any one of claims 1 to 8, wherein the absorbent body comprises an additional absorbent core which is stacked in the thickness direction in a region overlapping the narrow part in the crotch region of the absorbent core in the thickness direction and which has an average basis weight lower than the absorbent core.

10. The absorbent body according to any one of claims 1 to 9, wherein the absorbent core is partitioned in order from a front, into six equal parts including a first region through a sixth region, the abdomen-side region is constituted by the first region, the crotch region is constituted by the second region and the third region, and the back-side region is constituted by a fourth region through the sixth region.

11. A method of producing the absorbent body according to claim 1, comprising:

a preparation step of preparing a mold member comprising an abdomen-side part, a crotch part, and a back-side part and a narrowed part and a plurality of crotch part ridges, which respectively correspond to the abdomen-side region, the crotch region, and the back-side region and the narrow part and the plurality of crotch region grooves of the absorbent core,

wherein in the mold member, each of the abdomen-side part, the crotch part, and the back-side part has a plurality of openings, each of the plurality of ridges has no openings, and an opening ratio of the crotch part is higher than an opening ratio of each of the abdomen-side part and the back-side part, and
an absorbent core formation step of sprinkling pulp fibers and super absorbent polymer particles from one side of the mold member while reducing pressure from the other side of the mold member to deposit the pulp fibers and the super absorbent polymer particles on the mold member and form the absorbent core.

12. A method of producing the absorbent body according to claim 8, comprising:

a preparation step of preparing a mold member comprising an abdomen-side part, a crotch part, and a back-side part and a narrowed part, a plurality of crotch part ridges, and a plurality of crotch part sub-ridges, which respectively correspond to the abdomen-side region, the crotch region, and the back-side region and the narrow part, the plurality of crotch region grooves, and the plurality of crotch region sub-grooves of the absorbent core, wherein in the mold member, each of the abdomen-side part, the crotch part, and the back-side part has a plurality of openings, each of the plurality of crotch part ridges and the plurality of crotch part sub-grooves has no openings, and an opening ratio of the crotch part is higher than an opening ratio of each of the abdomen-side part and the back-side part, and
an absorbent core formation step of sprinkling pulp fibers and super absorbent polymer particles from one side of the mold member while reducing pressure from the other side of the mold member to deposit the pulp fibers and the super absorbent polymer particles on the mold member and form the absorbent core.

13. The method according to claim 11 or 12, wherein the abdomen-side part and the back-side part comprise a plurality of abdomen-side ridges and a plurality of back-side ridges, respectively, extending in the longitudinal direction, and a length in the width direction of each of the plurality of ridges is shorter than a length in the width direction of each of the plurality of abdomen-side ridges and the plurality of back-side ridges.

Fig. 1

Fig. 2

L/FD ←——→ L/RD

W

7

9  15

EP 4 574 116 A1

Fig. 3

L/FD ← → L/RD

W

9

45  47  43  41  35  41  IV→  43  WA  49  55  51  11

LA — — — — — — — — — — — — — — — — — — — — — — — — LA

53

IV→

| 23 | 25 | 27 | 29 | 31 | 33 |

| 17 | 19 | 21 |

WA

EP 4 574 116 A1

# Fig. 4

EP 4 574 116 A1

Fig. 5

EP 4 574 116 A1

Fig. 6

EP 4 574 116 A1

Fig. 7

Fig. 8

Fig. 9

Fig. 10

Fig. 11

## INTERNATIONAL SEARCH REPORT

| International application No. |
|---|
| **PCT/JP2022/036806** |

| A. | CLASSIFICATION OF SUBJECT MATTER |
|---|---|

*A61F 13/15*(2006.01)i; *A61F 13/532*(2006.01)i
FI:    A61F13/532 100; A61F13/15 321

According to International Patent Classification (IPC) or to both national classification and IPC

| B. | FIELDS SEARCHED |
|---|---|

Minimum documentation searched (classification system followed by classification symbols)

A61F13/15; A61F13/532

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2007-268253 A (KAO CORP.) 18 October 2007 (2007-10-18)<br>paragraphs [0016], [0017], [0059], [0060], [0078], [0079], fig. 2, 6 | 1-13 |
| Y | WO 2022/004499 A1 (UNI-CHARM CO., LTD.) 06 January 2022 (2022-01-06)<br>paragraphs [0028]-[0030], [0038], [0042], [0065], [0066], fig. 3, 4, 9, 10 | 1-13 |
| Y | JP 2006-149453 A (DAIO PAPER CORP.) 15 June 2006 (2006-06-15)<br>paragraph [0070], fig. 4 | 1-13 |
| Y | JP 10-502835 A (THE PROCTER & GAMBLE CO.) 17 March 1998 (1998-03-17)<br>fig. 6, 7 | 9-13 |
| A | JP 2012-11235 A (KAO CORP.) 19 January 2012 (2012-01-19) | 1-13 |
| A | JP 2019-526408 A (ACCUSENTRY INC.) 19 September 2019 (2019-09-19) | 1-13 |
| A | JP 2-501035 A (NORDSON CORP.) 12 April 1990 (1990-04-12) | 1-13 |

☐ Further documents are listed in the continuation of Box C.      ☑ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 November 2022** | **29 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/036806**

| Patent document cited in search report | | | Publication date (day/month/year) | Patent family member(s) | | | Publication date (day/month/year) |
|---|---|---|---|---|---|---|---|
| JP | 2007-268253 | A | 18 October 2007 | US | 2007/0208319 | A1 | |
| | | | | paragraphs [0024], [0025], [0072], [0073], [0100], [0101], fig. 2, 6 | | | |
| | | | | CN | 101032437 | A | |
| WO | 2022/004499 | A1 | 06 January 2022 | JP | 2022-12063 | A | |
| JP | 2006-149453 | A | 15 June 2006 | US | 2008/0038504 | A1 | |
| | | | | paragraph [0112], fig. 12 | | | |
| | | | | EP | 1772126 | A1 | |
| | | | | CN | 1976663 | A | |
| JP | 10-502835 | A | 17 March 1998 | US | 5873867 | A | |
| | | | | fig. 6, 7 | | | |
| | | | | WO | 1996/001608 | A1 | |
| | | | | EP | 692232 | A1 | |
| | | | | CN | 1151689 | A | |
| JP | 2012-11235 | A | 19 January 2012 | (Family: none) | | | |
| JP | 2019-526408 | A | 19 September 2019 | US | 2018/0061038 | A1 | |
| | | | | WO | 2018/039535 | A1 | |
| JP | 2-501035 | A | 12 April 1990 | US | 4927346 | A | |
| | | | | WO | 1988/004165 | A1 | |
| | | | | CN | 87108232 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018000872 A, Kokai
- JP 2021053237 A, Kokai
- JP 2019118722 A, Kokai